# EUROPEAN PATENT APPLICATION

(11) **EP 1 188 376 A1**
(43) Date of publication of application: **20.03.2002**
(21) Application number: 00940797.4
(22) Date of filing: 22.06.2000
(51) Int. Cl.: A01N 43/18, C07D 335/06

(54) **TRIKETONE DERIVATIVES AND HERBICIDES FOR LOWLAND FIELDS**

(30) Priority: 23.06.1999 JP 17756199
(71) Applicant: Idemitsu Kosan Co., Ltd., Tokyo 100-0005 (JP)
(72) Inventor: SAITOU, Masatoshi, Sodegaura-shi, Chiba 299-0205 (JP); SEKIGUCHI, Hiroki, Sodegaura-shi, Chiba 299-0205 (JP); OGAWA, Shin-ichiro, Sodegaura-shi, Chiba 299-0205 (JP)
(74) Representative: Gille Hrabal Struck Neidlein Prop Roos
(86) International application number: JP0004085
(87) International publication number: WO0078146

(57) **Abstract**

Triketone derivatives represented by the following formula (I) and salts thereof, 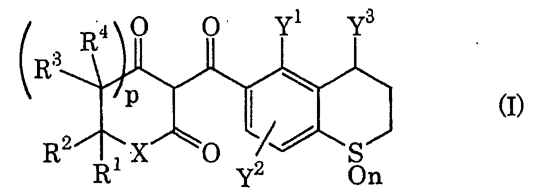 wherein Y¹, Y², Y³, n, p, R¹, R², R³ and R⁴ are as defined in the disclosure, and herbicidal compositions for paddy fields containing at least one of these compounds. The triketone derivatives and the herbicidal compositions for paddy fields are less injurious to paddy rice and have a broad spectrum of control against paddy field weeds with small application rates.

## Description

### Technical Field

The present invention relates to triketone derivatives and herbicidal compositions for paddy fields containing triketone derivatives.

### Background Art

Since herbicides are chemicals important for reducing labor of controlling weeds noxious to growth of cultivated crops and for achieving high productivity of agricultural and horticultural crops, efforts have been directed for a long time to development of new safe herbicides which exhibit high herbicidal effects in small application rates.

Recently, it is reported that a herbicidal composition containing as an effective component a triketone derivative having a bicyclic benzoyl structure is highly safe for upland crops and highly active for controlling upland weeds. For example, herbicidal compositions containing, as effective components, compounds disclosed in Japanese Patent No. 2579663 (United States Patent No. 5,098,464) and WO 97/08164 are excellent in controlling upland weeds. However, the reported herbicidal compositions are still insufficient in herbicidal effect against paddy field weeds and also insufficient in safety for paddy rice.

Therefore, an object of the present invention is to provide triketone derivatives and herbicidal compositions having a broad spectrum of controlling paddy field weeds in small application rates without causing injury to paddy rice.

### Disclosure of Invention

As a result of extensive studies to achieve the above object, the inventors have found that triketone derivatives represented by the formula (I) and their salts prevent growth of various paddy field weeds in small application rates without causing injury to paddy rice. The present invention has been accomplished based on this finding.

Thus, the present invention relates to:
(1) A herbicidal composition for paddy fields, containing a herbicidally effective amount of a triketone derivative represented by the following formula (I) and/or a herbicidally suitable salt thereof: 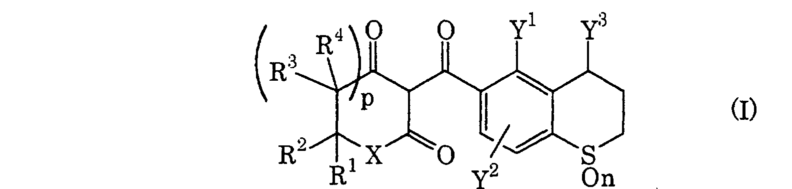 wherein Y¹ represents C₁-C₄ alkyl, halogen, C₁-C₄ haloalkyl, C₁-C₄ alkoxy, C₁-C₄ alkylthio, C₁-C₄ alkylsulfonyl, C₁-C₄ alkylamino, (C₁-C₄ alkyl)amido, nitro, amino, hydroxy, mercapto or cyano; Y² represents hydrogen, C₁-C₄ alkyl or halogen; Y³ represents hydrogen or C₁-C₆ alkyl; n represents 0, 1 or 2; p represents 0 or 1; R¹, R², R³ and R⁴ are each independently hydrogen, C₁-C₄ alkyl or phenyl, R¹ or R² being optionally bonded to R³ or R⁴ to form a double bond in the molecule when p is 1; and X represents an oxygen group atom or a group represented by the following formula: 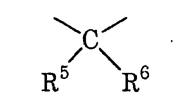 wherein R⁵ and R⁶ are each independently hydrogen, C₁-C₄ alkyl or phenyl.
(2) The herbicidal composition as described in (1), wherein Y¹ represents C₁-C₄ alkyl, halogen or C₁-C₄ haloalkyl.
(3) The herbicidal composition as described in (1) and (2), wherein Y¹ represents C₁-C₄ alkyl or halogen.
(4) The herbicidal composition as described in (1) to (3), wherein Y¹ represents methyl or chlorine.
(5) The herbicidal composition as described in (1) to (4), wherein n is 2.
(6) The herbicidal composition as described in (1) to (5), wherein X is a group represented by the following formula: 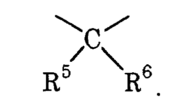
(7) The herbicidal composition as described in (1) to (6), wherein R⁵ and R⁶ are each hydrogen or methyl.
(8) The herbicidal composition as described in (1) to (7), wherein Y³ is hydrogen.
(9) The herbicidal composition as described in (1) to (8), wherein p is 1.
(10) The herbicidal composition as described in (1) to (9), wherein Y² is hydrogen, methyl or fluorine.

### Best Mode for Carrying Out the Invention

The present invention will be described below in more detail.

The triketone derivatives for use in the herbicidal compositions of the present invention will be described first.

The triketone derivatives of the present invention are represented by the formula (I):

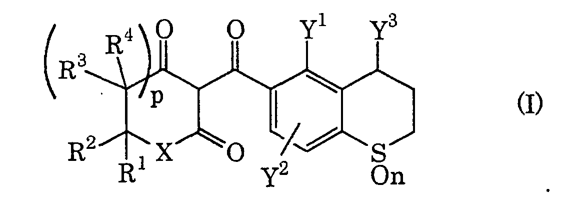

In the formula (I), Y¹ represents C₁-C₄ alkyl, halogen, C₁-C₄ haloalkyl, C₁-C₄ alkoxy, C₁-C₄ alkylthio, C₁-C₄ alkylsulfonyl, C₁-C₄ alkylamino, (C₁-C₄ alkyl)amido, nitro, amino, hydroxy, mercapto or cyano. Examples of C₁-C₄ alkyl include methyl, ethyl, propyls such as n-propyl and i-propyl and butyls such as n-butyl, s-butyl, t-butyl and i-butyl. Examples of halogen include fluorine, chlorine, bromine and iodine. Examples of C₁-C₄ haloalkyl include the above alkyls substituted with 1 to 9 halogen atoms such as -CH₂Cl, -CHCl₂, -CCl₃, -CH₂F, -CHF₂, -CF₃, -CCl₂CH₃, -CF₂CH₃, -CH₂CH₂F, -CH₂CH₂CHF₂, -CF₂CF₃, -CH₂CH₂CHCl₂, -CH₂CH₂CH₂CH₂Cl, -CH₂CH₂CH₂CH₂F, -CH(CH₃)CH₂Cl, -CH(CH₃)CH₂F and -CH(C₂H₅)CH₂F. Examples of C₁-C₄ alkoxy include methoxy, ethoxy, i-propoxy, n-propoxy, t-butoxy, i-butoxy, s-butoxy, and n-butoxy. Examples of C₁-C₄ alkylthio include methylthio, ethylthio, i-propylthio, n-propylthio, t-butylthio, i-butylthio and n-butylthio. Examples of C₁-C₄ alkylsulfonyl include methylsulfonyl, ethylsulfonyl, i-propylsulfonyl, n-propylsulfonyl, t-butylsulfonyl, i-butylsulfonyl, s-butylsulfonyl and n-butylsulfonyl. Examples of C₁-C₄ alkylamino include -NHCH₃, -N(CH₃)₂, -NHC₂H₅, -N(C₂H₅)₂ and -NCH₃(C₂H₅). Examples of (C₁-C₄ alkyl)amido include -NHCOCH₃, -NHCOC₂H₅, -NHCOC₃H₇ and -NHCOC4H9. Among the above, methyl and chlorine are preferable as Y¹.

Y² represents hydrogen, C₁-C₄ alkyl or halogen. Examples of C₁-C₄ alkyl and halogen are the same as those for Y¹. Y² is preferably hydrogen, methyl or fluorine and more preferably hydrogen or methyl. Y² may be bonded to either 7-position or 8-position, preferably 8-position of the thiochroman ring when it is C₁-C₄ alkyl or halogen.

Y³ represents hydrogen or C₁-C₆ alkyl. Examples of C₁-C₆ alkyl include methyl, ethyl, propyls such as n-propyl and i-propyl, butyls such as n-butyl and i-butyl, pentyls such as n-pentyl and hexyls such as n-hexyl. Hydrogen, methyl and ethyl are preferable, and hydrogen is more preferable as Y³.

Subscript "n" represents the number of oxygen bonded to sulfur atom, and is an integer of 0, 1 or 2. Sulfur constitutes sulfide when n is 0, sulfoxide when n is 1, and sulfone when n is 2. The subscript "n" is preferably 2, i.e., sulfone is preferable.

Subscript "p" represents an integer of 0 or 1. The (hetero)cyclic diketone ring bonded to the thiochroman ring is a five-membered ring when p is 0 and a six-membered ring when p is 1. The subscript "p" is preferably 1, i.e., the (hetero)cyclic diketone ring bonded to the thiochroman ring is preferably six-membered ring.

R¹, R², R³ and R⁴ are each independently hydrogen, C₁-C₄ alkyl or phenyl. Examples of C₁-C₄ alkyl are the same as those for Y¹. When p is 1, R¹ or R² may be bonded to R³ or R⁴ to form a double bond in the molecule. R¹, R², R³ and R⁴ are each preferably hydrogen or methyl and more preferably hydrogen.

X represents oxygen group atom or a group represented by the following formula:

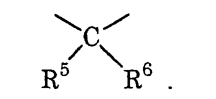

Examples of oxygen group atom include oxygen and sulfur. R⁵ and R⁶ in the alkylidene group represented by the above formula are each independently hydrogen, C₁-C₄ alkyl or phenyl. Examples of C₁-C₄ alkyl are the same as those for Y¹. X is preferably a group represented by the following formula: 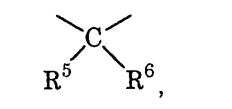 more preferably a group of the above formula wherein R⁵ and R⁶ are each independently hydrogen or methyl.

Preferred are triketone derivatives represented by the formula (I) wherein Y³ is hydrogen, n is 2, p is 1 and X is -CH₂-, -CH(CH₃)- or -C(CH₃)₂-, and more preferred are triketone derivatives represented by the formula (I) wherein Y¹ is chlorine or methyl, Y² is hydrogen, methyl or fluorine, Y³ is hydrogen, n is 2, p is 1, R¹ to R⁴ are each independently hydrogen or methyl, and X is -CH₂-, -CH(CH₃)- or -C(CH₃)₂-.

The triketone derivatives represented by the formula (I) may be tautomerized as shown below. The following tautomers are included in the ketone derivatives of the present invention. 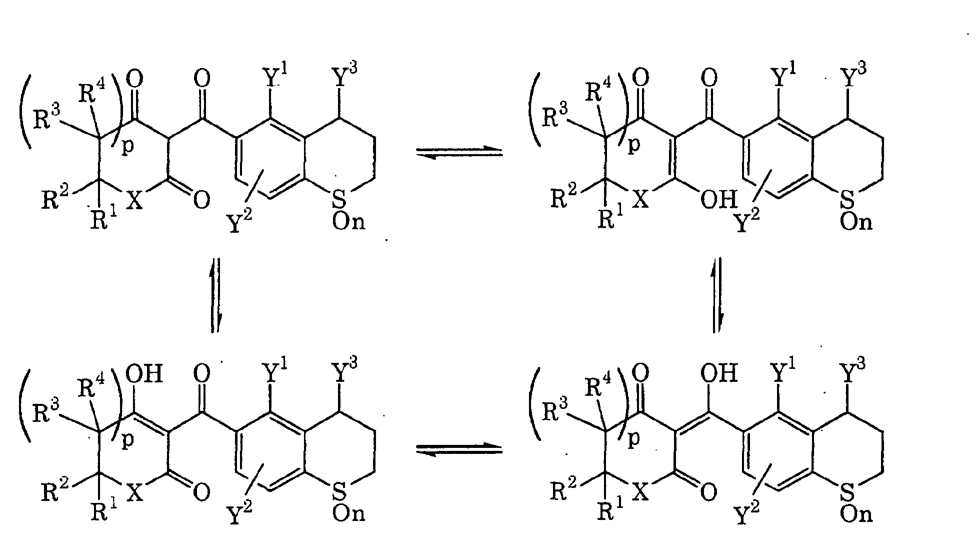 In the above formulae, Y¹, Y², Y³, n, p, R¹, R², R³, R⁴ and X are the same as defined in the formula (I).

The triketone derivatives represented by the formula (I) are acidic substances and can be easily made into salt forms by treating with a base. Such salts are also included in the present invention.

Known bases can be used without specific restriction. Examples of the base include organic bases such as amines and anilines and inorganic bases such as sodium compounds and potassium compounds. Examples of the amines include monoalkylamines, dialkylamines and trialkylamines. Alkyl in the alkylamines is generally C₁-C₄ alkyl. Examples of the anilines include aniline, monoalkylanilines and dialkylanilines. Alkyl in the alkylanilines is generally C₁-C₄ alkyl. Examples of the sodium compounds include sodium hydroxide and sodium carbonate. Examples of the potassium compounds include potassium hydroxide and potassium carbonate. Of the above bases, sodium compounds, potassium compounds and trialkylamines are preferable.

The triketone derivatives for use in herbicidal compositions for paddy fields of the present invention can be produced, for example, by the following process. 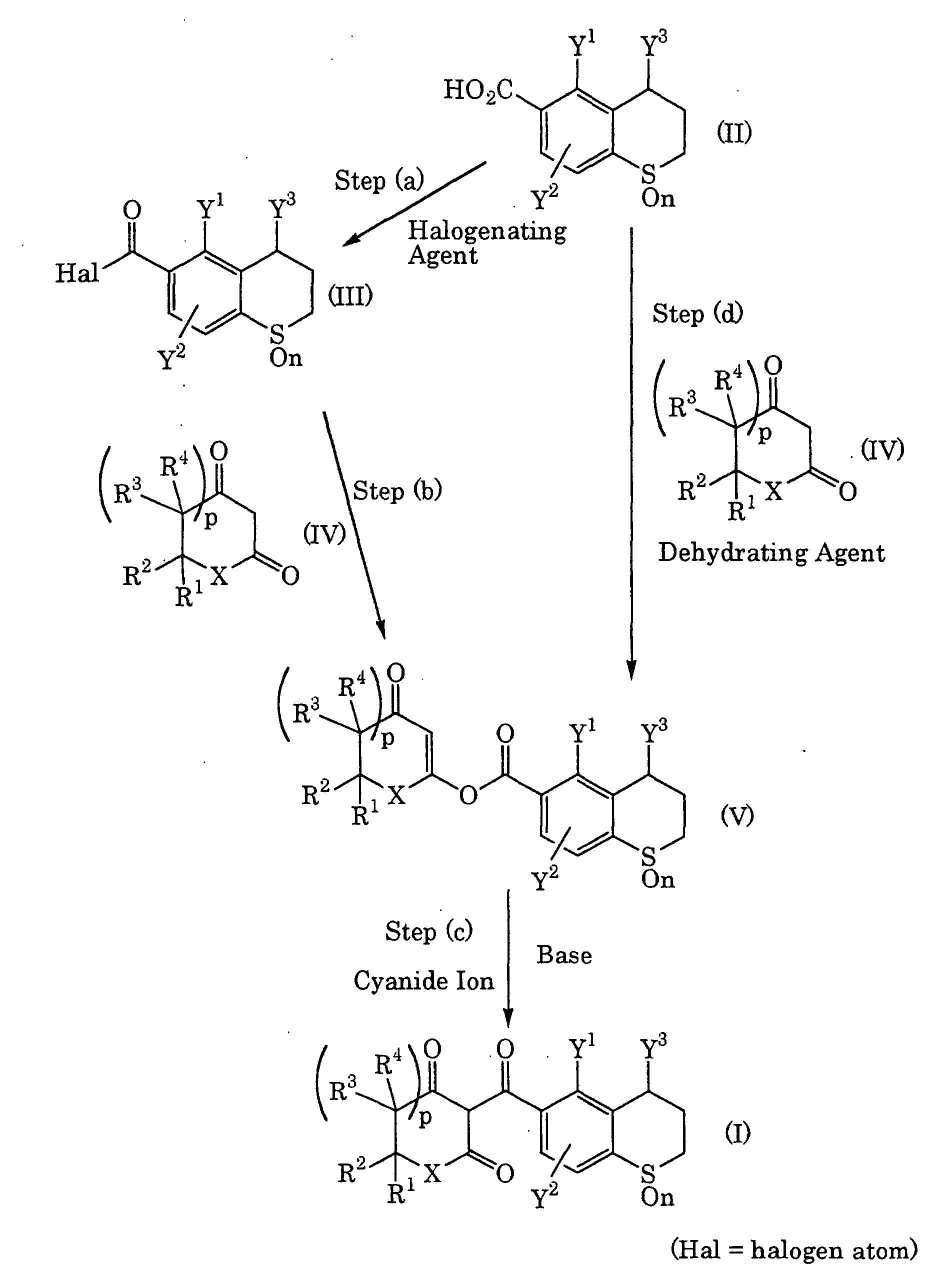 In the above formulae, Y¹, Y², Y³, n, p, R¹, R², R³, R⁴ and X are as defined above.

A compound represented by the formula (II) is reacted with a halogenating agent to prepare a compound.represented by the formula (III), which is then reacted with a compound represented by the formula (IV) to prepare a compound represented by the formula (V). Then, the compound (V) is rearranged into the triketone derivatives represented by the formula (I). The compound (V) may be also prepared by the reaction of the compound (II) with the compound (IV) in the presence of a dehydrating agent such as dicyclohexylcarbodiimide (hereinafter abbreviated as DCC).

Each step of the process will be described below in more detail.

### Step (a)

In the step (a), the compound (II) is reacted with a halogenating agent to form the compound (III). As the halogenating agent, for example, thionyl chloride, phosphorus oxychloride, etc. can be used. The halogenating agent is generally used in equimolecular amount or more to the compound (II). The reaction may be conducted in the presence or absence of a solvent. The solvent usable in the reaction is not particularly limited and may be inert solvent such as 1,2-dichloroethane and chloroform. An excess amount of thionyl chloride as the halogenating agent also serves as the solvent. The reaction temperature of the step (a) is not particularly limited, and preferably from 0°C to the refluxing temperature of the reaction system or the boiling point of the solvent and more preferably 60°C or its vicinity.

### Step (b)

In the step (b), the compound (III) obtained by the step (a) is reacted with the compound (IV) to form the compound (V). The compound (III) and the compound (IV) are reacted by mixing in a molar ratio of 1:1 to 1:3 generally in the presence of a solvent inert to the reaction. Examples of the solvent inert to the reaction include dioxane, acetonitrile, benzene, toluene, chloroform, methylene chloride and 1,2-dichloroethane. Two-phase solvents such as water -benzene, water-toluene and water-chloroform can be also used. To make the reaction proceed smoothly, a base such as sodium hydroxide, potassium hydroxide, sodium carbonate, potassium carbonate, triethylamine and pyridine may be used in equimolecular amount or more to the compound (III). The reaction temperature of the step (b) is not particularly limited, and preferably 0 to 60°C, more preferably 0°C to room temperature.

### Step (c)

In the step (c), the compound (V) obtained in the step (b) is rearranged into the triketone derivative represented by formula (I). The step (c) is conducted preferably in the presence of a solvent inert to the reaction such as methylene chloride, 1,2-dichloroethane, toluene, acetonitrile, N,N-dimethylformamide and ethyl acetate. Acetonitrile is particularly preferred. In the step (c), a base such as sodium carbonate, potassium carbonate, triethylamine and pyridine is added 1 to 4 times, preferably 1 to 2 times the equivalent amount to the compound (V). The step (c) is preferably carried out in the presence of hydrogen cyanide or a compound capable of generating cyanide anion in the reaction system, i.e., so called cyanide source so as to make the reaction proceed catalytically and smoothly. Examples of the cyanide source include metal cyanides such as sodium cyanide and potassium cyanide and cyanohydrin compounds of lower alkyl ketones, for example, cyanohydrins of about C₃-C₅ alkyl ketones such as acetone cyanohydrin and methyl isopropyl ketone cyanohydrin. When a metal cyanide is used, it is preferred to add a phase-transfer catalyst such as crown ethers to the reaction system so as to make the reaction proceed more smoothly. Hydrogen cyanide or the cyanide source is used generally 0.01 to 0.5 molar equivalent, preferably 0.05 to 0.2 molar equivalent to the compound (V). The reaction temperature of the step (c) is not particularly limited, and generally 0 to 80°C, preferably 20 to 40°C.

### Step (d)

The compound (V) can be obtained also by the step (d). In the step (d), the condensation reaction of the compound (II) and the compound (IV) is conducted in the presence of a solvent and a dehydrating agent such as DCC, thereby forming the compound (V). The solvent used in the condensation reaction is not particularly limited as long as the solvent is inert to the reaction. In general, acetonitrile or tert-amyl alcohol is preferably used. The reaction temperature of the step (d) is not particularly limited as long as within the range of 0°C to the boiling point of the solvent, and is preferably around room temperature. Usable dehydrating agents other than DCC may be 1,1-carbonyldiimidazole (CDI) and 1-(3-dimethylaminopropyl)-3-ethylcarbodiimide (EDC). The dehydrating agent is used in 1.0 to 3.0 equivalent and preferably 1.0 to 1.5 equivalent to the compound (II). The compound (II) and the compound (IV) are mixed in a molar ratio (compound (II) / compound (IV)) of 1:1 to 1:3 and preferably 1:1 to 1:1.5. The condensation reaction is completed generally in about 8 hours. However, the reaction time can be suitably selected from 1 to 48 hours, preferably from 5 to 15 hours depending on kinds of the compounds (II) and (V).

The compound (V) obtained by the steps (a) and (b) or the step (d) is treated in the following step (c) generally after isolation. However, the compound (V) may be treated without isolation.

The compound (II) which is used as the starting material in the production of the triketone derivatives for use in herbicidal compositions of the present invention can be prepared by the method described in USP 5,607,898 or modified methods thereof. The compounds of the formula (IV) are mostly known or easily produced by known methods.

Preferred triketone derivatives (I) for use in herbicidal compositions of the present invention are listed in Table 1.

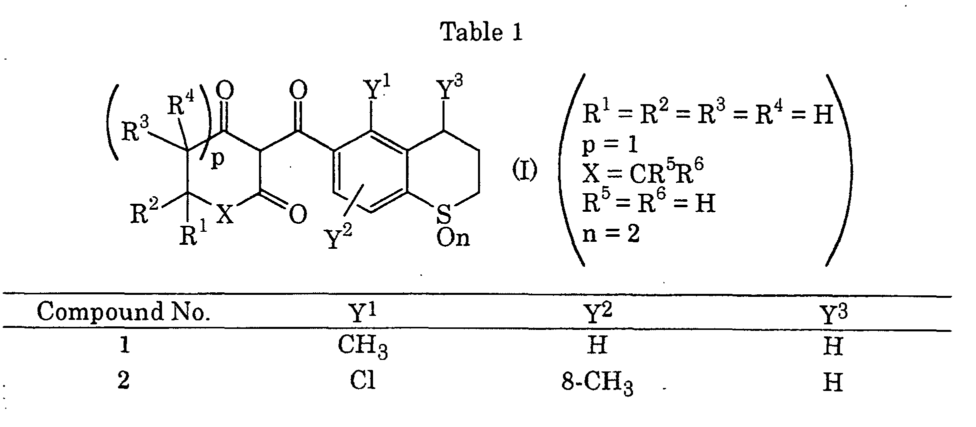

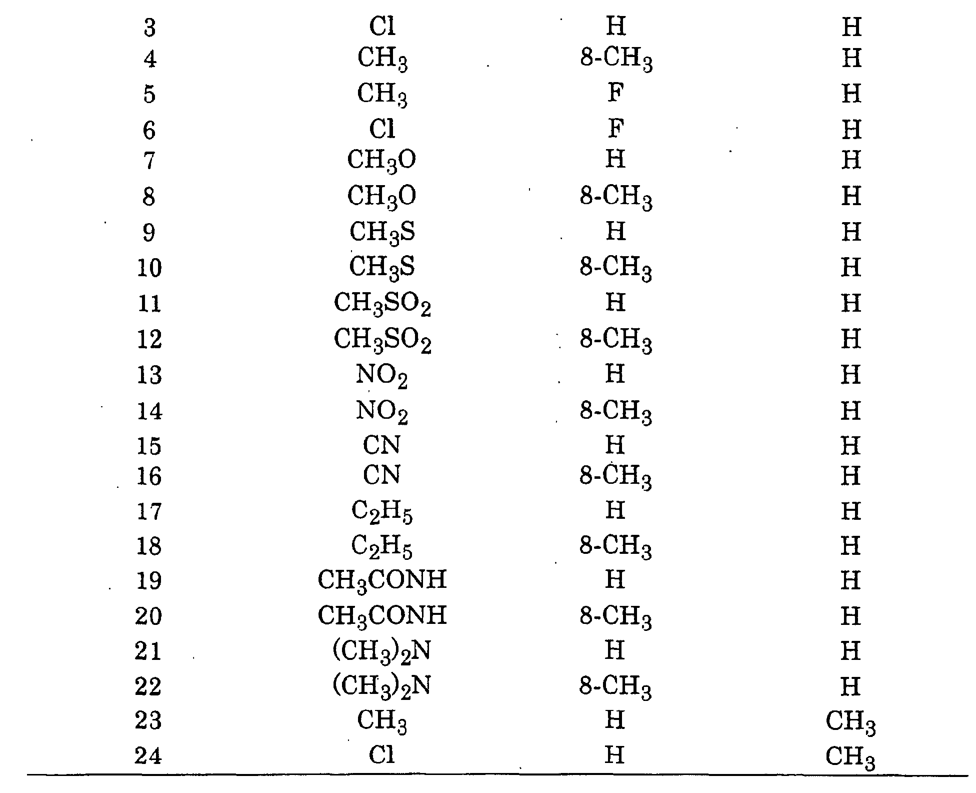

In Table 1, "8-CH₃" in the column entitled Y² means that methyl is attached to 8-position of the thiochroman ring.

Next, the herbicidal compositions for paddy fields of the present invention will be described.

The herbicidal compositions for paddy fields of the present invention contain the triketone derivatives of the formula (I) and/or salts thereof as the effective components. The triketone derivatives and their salts are mixed with a liquid carizer such as solvent or a solid carrier such as fine mineral powder to prepare formulations such as wettable powders, emulsifiable concentrates, dusts and granules. A surfactant is preferably added to improve emulsifying property, dispersing property and spreading property during the mixing process of the triketone derivatives and the carriers.

As the solvent serving as the liquid carrier, organic solvents are generally used. Specifically, the solvent may be aromatic hydrocarbons such as benzene, toluene and xylene; chlorinated hydrocarbons such as o-chlorotoluene, trichloroethane and trichloroethylene; alcohols such as cyclohexanol, amyl alcohol and ethylene glycol; ketones such as isophorone, cyclohexanone and cyclohexenylcyclohexane; ethers such as butylcellosolve, diethyl ether and methyl ethyl ether; esters such as isopropyl acetate, benzyl acetate and methyl phthalate; amides such as dimethylformamide; and mixtures thereof.

The fine powder of minerals are used as the solid carrier, which may be powders of oxides such as diatomaceous earth and slaked lime; powders of phosphates such as apatite; powders of sulfates such as gypsum and powders of silicates such as talc, pyroferrite, clay, kaolin, bentnite, acidic white clay, white carbon, quartz and silica.

As the surfactant, usable are any of anionic surfactants such as alkyl sulfates, alkylbenzenesulfonates, dialkyl sulfosuccinates and condensates of naphthalenesulfonic acid and form aldehyde; nonionic surfactants such as polyoxyethylene alkyl ethers, polyoxyethylene alkylphenol ethers, polyoxyethylene alkylamines and sorbitan esters of fatty acids; cationic surfactants such as alkylamine salts of fatty acids and quaternary ammonium salts; and amphoteric surfactants such as amino acids and betaine.

Herbicidal compositions of wettable powder formulation can be prepared, in general, by blending 10 to 55% by weight of the triketone derivative, 40 to 88% by weight of the solid carizer and 2 to 5% by weight of the surfactant. Emulsifiable concentrates can be prepared, in general, by mixing 20 to 50% by weight of the triketone derivative, 35 to 75% by weight of the solvent and 5 to 15% by weight of the surfactant. Dusts can be prepared, in general, by blending 1 to 15% by weight of the triketone derivative, 80 to 97% by weight of the solid carrier and 2 to 5% by weight of the surfactant. Granules can be prepared, in general, by blending 1 to 15% by weight of the triketone derivative, 80 to 97% by weight of the solid carrier and 2 to 5% by weight of the surfactant.

In addition to the triketone derivatives of the formula (I), the herbicidal compositions for paddy fields of the present invention may further contain, if necessary, another herbicidally active component known in the art, which may suitably selected from phenoxy herbicides, diphenyl ether herbicides, triazine herbicides, urea herbicides, carbamate herbicides, thiol carbamate herbicides, acid anilide herbicides, pyrazole herbicides, phosphoric acid herbicides, sulfonylurea herbicides and oxadiazine herbicides.

The herbicidal compositions for paddy fields of the present invention may further contain, if necessary, insecticide, antibiotic, plant growth regulator and fertilizer.

The herbicidal compositions of the present invention are applied to weeds before and/or after germination or their environments. The modes of application vary depending on the type of paddy rice species and the environmental factors. For example, the herbicidal compositions may be applied by spraying, scattering, sprinkling or irrigating.

The application rate of the triketone derivatives of the present invention is determined by a number of factors such as formulation selected, mode of application, amount and type of weed species, growing conditions, etc. In general, the application rate is 0.001 to 10 kg/ha, preferably 0.01 to 5 kg/ha. One skilled in the art can easily determine the application rate necessary for the desired level of weed control.

Paddy weeds which can be controlled by the herbicidal compositions of the present invention may include Alismataceous weeds such as *Alisma canaliculatum*, *Sagittaria trifolia* and *Sagittaria pygmaea*; Cyperaceous weeds such as *Cyperus difformis, Cyperus serotinus*, *Scirpus juncoides* and *Eleocharis kuroguwai*; Scrophulariaceous weeds such as *Lindernia pyxidaria*; Pontenderiaceous weeds such as *Monochoria vaginalis*; Potamogetonaceous weeds such as *Potamogeton distinctus*; Lythraceous weeds such as *Rotala indica*; and Gramineous weeds such as *Echinochloa crug-galli.*

The present invention will be described in further detail by way of the following Examples which are not intended to limit the scope of the invention thereto.

### Preparation Example 1

### Synthesis of 6-(1,3-dioxycyclohexan-2-yl)-5-methylthiochroman 1,1-dioxide (Compound No. 1)

Into a suspension of 0.80 g (3.3 mmol) of 5-methylthiochroman-6-carboxylic acid 1,1-dioxide in 4 ml of 1,2-dichloroethane, 0.29 ml (1.2 eq, 4.0 mmol) of thionyl chloride was added and heated for one hour and 30 minutes under refluxing. Excess thionyl chloride and 1,2-dichloroethane were evaporated off under reduced pressure, followed by addition of 1.6 ml acetonitrile. After cooling the resultant mixture in ice water bath, a solution prepared by dissolving 0.44 g (1.2 eq, 3.9 mmol) of 1,3-cyclohexanedione and 0.55 ml (1.2 eq, 3.9 mmol) of triethylamine in 2.4 ml of acetonitrile was added dropwise to the mixture, which was then stirred for 30 minutes in the ice water bath and further stirred for two hours while allowing the temperature to return to room temperature. Then, 0.55 ml (1.2 eq, 3.9 mmol) of triethylamine and 0.10 ml (0.33 eq, 1.1 mmol) of acetone cyanohydrin were added to the reaction solution, followed by stirring for three hours at 40°C. After evaporating off acetonitrile under reduced pressure, ice water and 10 ml of aqueous 10% sodium hydroxide were added to the mixture, which was then washed with methylene chloride. After adding 20 ml of concentrated hydrochloric acid, the reaction solution was extracted with methylene chloride twice and dried over anhydrous sodium sulfate. After evaporating off the solvent under reduced pressure, 0.88 g (79% yield) of 6-(1,3-dioxycyclohexan-2-yl)-5-methylthiochroman 1,1-dioxide was obtained. The results of ¹H-NMR (nuclear magnetic resonance) analysis and IR (infrared) analysis are shown below:
¹H-NMR (CDCl₃): 1.9-3.1 (m, 10H), 2,14 (s, 3H), 3.2-3.5 (m, 2H), 7.08 (d, 1H), 7.82 (d, 1H)
IR (KBr): 3500, 2990, 1690,1550, 1300, 1135 cm⁻¹

### Preparation Examples 2 to 4

### Synthesis of Compounds Nos. 2 to 4

In the same manner as in Preparation Example 1 except that the corresponding carboxylic acids were used in place of 5-methylthiochroman-6-carboxylic acid 1,1-dixode, Compounds Nos. 2 to 4 were obtained. The results of ¹H-NMR analysis and IR analysis are shown in Table 2.

**Table 2**

| Compound No. | ¹H-NMR (ppm) Internal Standard: tetrahydrofuran Solvent: heavy chloroform | IR (cm⁻¹) KBr tablet method |
|---|---|---|
| 2 | 2.0-3.2(m, 10H), 2.73(s, 3H), 3.3-3.5(m, 2H), 6.96(s, 1H) | 3490, 2960, 1685, 1565, 1300, 1150 |
| 3 | 1.8-3.2(m, 10H), 3.3-3.5(m, 2H), 7.22(d, 1H), 7.83(d, 1H) | 3500, 2955, 1680, 1560, 1315, 1145 |
| 4 | 2.0-3.0(m,10H), 2.08(s, 3H), 2.71(s, 3H), 3.3-3.5(m, 2H), 6.84(s, 1H) | 3450, 2950, 1680, 1560, 1295, 1125 |

### Examples 1-4 and Comparative Examples 1-4

### [1] Preparation of herbicidal compositions

A carrier for wettable powder was prepared by pulverizing and mixing uniformly 57 parts by weight of talc and 40 parts by weight bentonite as carriers and 3 parts by weight of sodium alkylbenzenesulfonate.

Each herbicidal composition was prepared by pulverizing and uniformly mixing 90 parts by weight of the carrier for wettable powder and 10 parts by weight of each compound of the invention. In the same manner, herbicidal compositions were prepared as Comparative Examples 1 to 4 using the following compound Nos. 5 to 8.

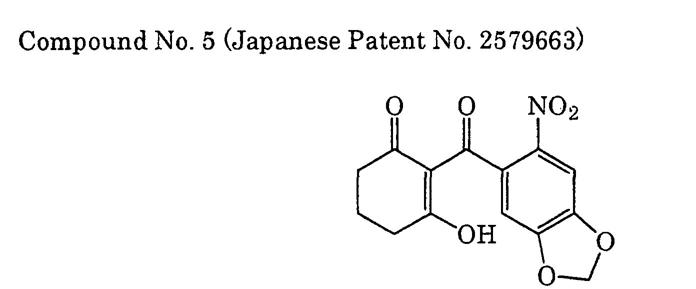

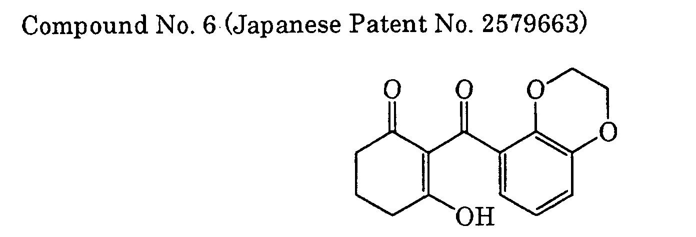

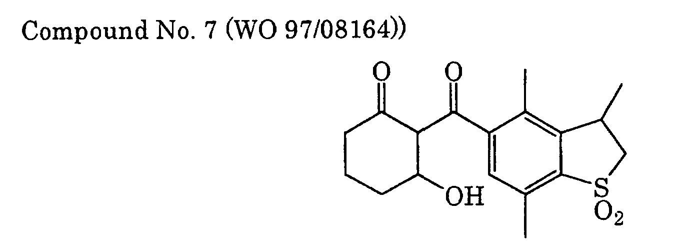

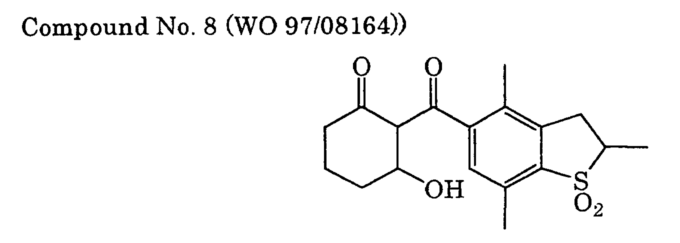

### [2] Herbicidal tests

### (1) Test-1 (Irrigation test/treatment three days after transplantation)

Seeds of *Echinochloa oryzicola* and *Scirpus juncoides* were sowed on the surface of paddy field soil in a Wagner pot of 1/2000 are. Then, rice seedlings of 2.5 leaf stage were transplanted in the soil.

After putting water into the pot so as to have an irrigation water depth of 3 cm, the pot was kept in a green house controlled at 20 to 25°C under conditions for rice vegetation.

Three days after the transplantation of rice seedlings, an amount of the herbicidal composition prepared in [1] was added to irrigation water. The herbicidal effect and injury to paddy rice were determined 30 days after the treatment with the herbicidal composition. The results are shown in Table 3.

### (2) Test-2 (Irrigation test/treatment ten days after transplantation)

Seeds of *Echinochloa oryzicola* and *Scirpus juncoides* were sowed on the surface of paddy field soil in a Wagner pot of 1/2000 are. Then, rice seedlings of 2.5 leaf stage were transplanted in the soil.

After putting water into the pot so as to have an irrigation water depth of 3 cm, the pot was kept in a green house controlled at 20 to 25°C under conditions for rice vegetation.

Ten days after the transplantation of rice seedlings, an amount of the herbicidal composition prepared in [1] was added to irrigation water. The herbicidal effect and injury to paddy rice were determined 30 days after the treatment with the herbicidal composition. The results are shown in Table 3.

The herbicidal effect and the degree of injury were determined by the following ratings.

### (a) Herbicidal effect

| [Ratings of herbicidal effect] | [Degree of control (weed control ratio)] |
|---|---|
| 0 | smaller than 5% (almost no effect) |
| 1 | 5% to smaller than 20% |
| 2 | 20% to smaller than 40% |
| 3 | 40% to smaller than 70% |
| 4 | 70% to smaller than 90% |
| 5 | 90% or greater (almost complete control) |

### (b) Injury

| [Ratings of injury] | [Observed injury] |
|---|---|
| 0 | no injury |
| 1 | almost no injury |
| 2 | slight injury |
| 3 | significant injury |
| 4 | marked injury |
| 5 | almost complete death |

**Table 3**

| | Compound No. | Application rate (g/ha) | Treatment three days after transplantation | | | Treatment ten days after transplantation | |
|---|---|---|---|---|---|---|---|
| | | | Herbicidal effect | | Injury | Herbicidal effect | |
| | | | A* | B* | C* | A* | B* |
| Examples | | | | | | | |
| 1 | 1 | 100 | 3 | 5 | 0 | 3 | 4 |
| 2 | 2 | 100 | 4 | 5 | 1 | 4 | 5 |
| 3 | 3 | 100 | 4 | 5 | 0 | 4 | 5 |
| 4 | 4 | 100 | 3 | 5 | 0 | 3 | 4 |

| Comparative Examples | | | | | | | |
|---|---|---|---|---|---|---|---|
| 1 | 5 | 100 | 5 | 4 | 3 | 0 | 1 |
| 2 | 6 | 100 | 0 | 0 | 0 | 0 | 0 |
| 3 | 7 | 100 | 4 | 3 | 3 | 3 | 2 |
| 4 | 8 | 100 | 3 | 3 | 2 | 1 | 1 |

| | | | | | | | |
|---|---|---|---|---|---|---|---|
| ^{A}**Echinochloa oryzicola* | | | | | | | |
| ^{B}**Scirpus juncoides* | | | | | | | |
| ^{C}*Paddy rice. | | | | | | | |

### Industrial Applicability

The triketone derivatives and herbicidal compositions for paddy fields of the present invention are less injurious to paddy rice and have a broad spectrum of weed control with small application rates.

## Claims

1. A herbicidal composition for paddy field weeds, containing a herbicidally effective amount of a triketone derivative represented by the following formula (I) and/or a herbicidally acceptable salt thereof: 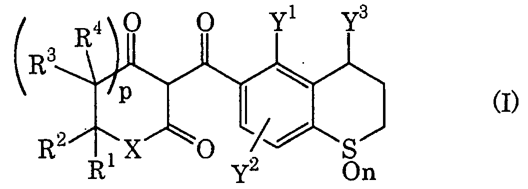 wherein
Y¹ represents C₁-C₄ alkyl, halogen, C₁-C₄ haloalkyl, C₁-C₄ alkoxy, C₁-C₄ alkylthio, C₁-C₄ alkylsulfonyl, C₁-C₄ alkylamino, (C₁-C₄ alkyl)amido, nitro, amino, hydroxy, mercapto or cyano;
Y² represents hydrogen, C₁-C₄ alkyl or halogen;
Y³ represents hydrogen or C₁-C₆ alkyl;
n represents 0, 1 or 2;
p represents 0 or 1;
R¹, R², R³ and R⁴ each independently represents hydrogen, C₁-C₄ alkyl or phenyl, R¹ or R² being optionally bonded to R³ or R⁴ to form a double bond in the molecule when p is 1; and
X represents an oxygen group atom or a group represented by the following formula: 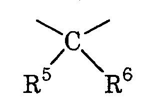
wherein R⁵ and R⁶ each independently represents hydrogen, C₁-C₄ alkyl or phenyl.

2. The herbicidal composition according to claim 1, wherein Y¹ represents C₁-C₄ alkyl, halogen or C₁-C₄ haloalkyl.

3. The herbicidal composition according to claim 1 or 2, wherein Y¹ represents C₁-C₄ alkyl or halogen.

4. The herbicidal composition according to any one of claims 1 to 3, wherein Y¹ represents methyl or chlorine.

5. The herbicidal composition according to any one of claims 1 to 4, wherein n is 2.

6. The herbicidal composition according to any one of claims 1 to 5, wherein X is the group represented by the following formula:

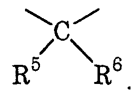

7. The herbicidal composition according to any one of claims 1 to 6, wherein R⁵ and R⁶ are each hydrogen or methyl.

8. The herbicidal composition according to any one of claims 1 to 7, wherein Y³ is hydrogen.

9. The herbicidal composition according to any one of claims 1 to 8, wherein p is 1.

10. The herbicidal composition according to any one of claims 1 to 9, wherein Y² is hydrogen, methyl or fluorine.

11. A triketone derivative represented by the following formula (I') or a salt thereof, 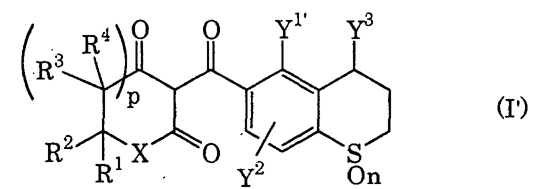 wherein
Y^{1'} represents C₁-C₄ alkoxy, C₁-C₄ alkylthio, C₁-C₄ alkylsulfonyl, C₁-C₄ alkylamino, (C₁-C₄ alkyl)amido, nitro, amino, hydroxy, mercapto or cyano; Y² represents hydrogen, C₁-C₄ alkyl or halogen;
Y³ represents hydrogen or C₁-C₆ alkyl;
n represents 0, 1 or 2;
p represents 0 or 1;
R¹, R², R³ and R⁴ each independently represents hydrogen, C₁-C₄ alkyl or phenyl, R¹ or R² being optionally bonded to R³ or R⁴ to form a double bond in the molecule when p is 1; and
X represents an oxygen group atom or a group represented by the following formula: 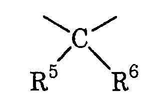
wherein R⁵ and R⁶ each independently represents hydrogen, C₁-C₄ alkyl or phenyl.

12. A method of controlling paddy field weeds, comprising contacting the paddy field weeds or their environment with a herbicidally effective amount of a triketone derivative and/or a salt thereof defined in claim 1.

13. The method according to claim 12, wherein Y¹ represents C₁-C₄ alkyl, halogen or C₁-C₄ haloalkyl.

14. The method according to claim 12 or 13, wherein Y¹ represents C₁-C₄ alkyl or halogen.

15. The method according to any one of claims 12 to 14, wherein Y¹ represents methyl or chlorine.

16. The method according to any one of claims 12 to 15, wherein n is 2.

17. The method according to any one of claims 12 to 16, wherein X is the group represented by the following formula:

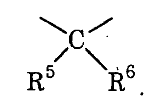

18. The method according to any one of claims 12 to 17, wherein R⁵ and R⁶ are each hydrogen or methyl.

19. The method according to any one of claims 12 to 18, wherein Y³ is hydrogen.

20. The method according to any one of claims 12 to 19, wherein p is 1.

21. The method according to any one of claims 12 to 20, wherein Y² is hydrogen, methyl or fluorine.
